(19) Europäisches Patentamt
European Patent Office
Office européen des brevets

(11) Publication number : **0 356 128 B1**

# (12) EUROPEAN PATENT SPECIFICATION

(45) Date of publication of patent specification : **01.07.92 Bulletin 92/27**

(51) Int. Cl.$^5$ : **A61K 31/66,** C07F 9/30, C07F 9/572

(21) Application number : **89308311.3**

(22) Date of filing : **16.08.89**

## (54) 3-(Aminopropyl)methyl phosphinic acid as a therapeutic agent.

(30) Priority : **26.08.88 GB 8820266**

(43) Date of publication of application : **28.02.90 Bulletin 90/09**

(45) Publication of the grant of the patent : **01.07.92 Bulletin 92/27**

(84) Designated Contracting States : **AT BE CH DE ES FR GB GR IT LI LU NL SE**

(56) References cited :
**EP-A- 0 181 833**
**DE-A- 2 032 712**

(73) Proprietor : **SMITH KLINE & FRENCH LABORATORIES LIMITED**
**Mundells**
**Welwyn Garden City Hertfordshire, AL7 1EY (GB)**

(72) Inventor : **Hills, Judith Mary**
**33 Church Street Isham**
**Nr. Kettering Northamptonshire NN14 1HD (GB)**
Inventor : **Howson, William**
**24 Horslow Street**
**Potton Bedfordshire SG19 2NX (GB)**

(74) Representative : **Giddings, Peter John, Dr. et al**
**Smith Kline & French Laboratories Ltd.**
**Corporate Patents Mundells**
**Welwyn Garden City Hertfordshire AL7 1EY (GB)**

EP 0 356 128 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to the use in therapy, in particular as selective $GABA_B$ receptor agonists, of certain phosphinic acid derivatives and pharmaceutical compositions containing said derivatives.

Compounds having activity at $GABA_B$ receptor sites are known in the art. For example EP-0181833-A1 (equivalent to US 4,656,298) discloses compounds of structure

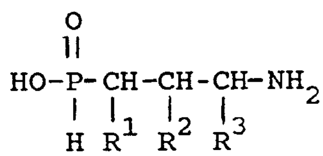

in which $R^1$ to $R^3$ can be, for example, hydrogen, $C_{1-8}$alkyl or phenyl, as having $GABA_B$ receptor agonist and antagonist properties and also mixed $GABA_A$ and $GABA_B$ receptor agonist properties. Compounds having activity as $GABA_B$ receptor agonists can be used as muscle relaxants, and compounds combining $GABA_B$ with $GABA_A$ receptor agonist properties may be used as antidepressants. Compounds having $GABA_B$ receptor antagonist properties are expected to act as muscle stimulants and be active in muscular atrophy and distrophy. In view of the variation in potential utilities depending on the precise nature of the affinity of compounds at the different receptor sites clearly there is a need for compounds having greater specificity for the different receptor sites.

It has now surprisingly been found that this need for highly specific (and potent) $GABA_B$ receptor agonists can be fulfilled by the compound, 3-(aminopropyl)methyl phosphinic acid, a compound which has previously only been described in the literature in the form of its sodium salt as a synthetic intermediate with no biological activity associated therewith (see US 3,970,586, 3,374,288 and GB 1351503).

The present invention therefore provides, in a first aspect, the use of a 3-(aminopropyl)methyl phosphinic acid of structure (I)

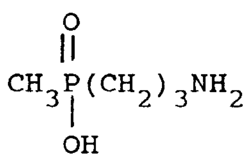

(I)

or a pharmaceutically acceptable salt thereof, in therapy.

Suitable pharmaceutically acceptable salts of the compound (I) include for example the corresponding acid addition salts. Suitable acids for the formation of such salts include, for example, mineral acids such as hydrochloric, hydrobromic, sulphuric or phosphoric acid, or organic acids such as organic sulphonic acids, for example, benzenesulphonic, p-toluenesulphonic or methansulphonic acid, and organic carboxylic acids, such as acetic, lactic, palmitic, stearic, maleic, maleic, fumaric, tartaric, ascorbic or citric acid. In addition salts may be formed with suitable metal ions such as sodium, potassium, lithium or magnesium ions.

It is to be noted, and will be appreciated by persons skilled in the art that under appropriate pH conditions the compound of structure (I) can exist in Zwitterionic form, i.e. as

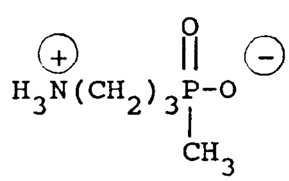

.

The compound of structure (I) and salts thereof can be prepared by processes known in the art, for example by,

(a) hydrolysis of a compound of structure (II)

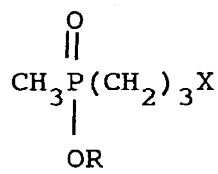

(II)

in which R is a protecting group and X is a protected amino group, or
(b) reduction of a compound of structure (III)

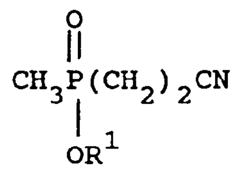

(III)

in which $R^1$ is hydrogen or a protecting group;
and optionally thereafter removing any protecting groups and forming a salt.

Suitable protected amino groups X will be apparent to those skilled in the art, and include, for example, acylamino groups such as acetylamino, phthalimido, benzyloxycarbonylamino or t-butyloxycarbonylamino or aryl$C_{1-4}$alkylamino groups e.g. benzylamino. A preferred protected amino group X is phthalimido.

Suitable protecting groups R will be apparent to those skilled in the art and include, for example $C_{1-4}$-alkyl, in particular ethyl and $C_{1-4}$aralkyl, in particular benzyl.

Suitable protecting groups $R^1$ will be apparent to those skilled in the art and include for example, $C_{1-4}$alkyl, in particular ethyl and trimethylsilyl.

Suitable conditions for hydrolysis of the compounds of structure (II) will be apparent to those skilled in the art. In particular, when X is phthalamido and R is ethyl, the deprotection can be carried out under aqueous conditions in the presence of hydrochloric acid and acetic acid at reflux temperature. Alternatively, when R is $C_{1-4}$aralkyl, for example benzyl, the deprotection can be carried out by hydrogenation in the presence of a suitable catalyst, for example platinum or palladium on carbon.

Suitable conditions for the reduction of compounds of structure (III) will be apparent to those skilled in the art and include, for example Raney nickel in a suitable solvent, such as a $C_{1-4}$alkanol, in particular ethanol and in the presence of ammonia. Protecting groups $R^1$ can be removed by standard techniques, for example if $R^1$ is ethyl by hydrolysis in the presence of a suitable acid such as hydrochloric acid.

The compounds of structure (II), which themselves are new can be prepared, for example, when in structure (II) X is phthalimido and R is Et, by reaction of diethyl methyl phosphonite (prepared according to Hoffman, F.W. and Moore T.R. JACS, 1958, <u>80</u>, 1150-1154) and N-(3-bromopropyl)phthalamide, in an organic solvent, in particular toluene at elevated temperature.

The compounds of structure (III) which are themselves novel can also be prepared by procedures analogous to those known in the art. For example, compounds of structure (III) in which $R^1$ is ethyl can be prepared by reaction of diethyl methyl phosphonite and 2-bromopropionitrile. Alternatively, compounds of structure (III) in which $R^1$ is trimethylsilyl can be prepared by reaction of methyl phosphinic acid with trimethylsilylchloride in the presence of a suitable base such as triethylamine, followed by reaction with acrylonitrile.

The compound of structure (I) has been found to be an extremely potent and selective agonist of $GABA_B$ receptor sites.

More specifically, the compound has been found in <u>in vitro</u> binding assays to be significantly more potent than the known $GABA_B$ agonist baclofen and, surprisingly, much more selective for the $GABA_B$ receptor than the $GABA_A$ receptor when compared to the known selective $GABA_B$ agonist 3-aminopropyl phosphinic acid disclosed in EP-0181833-A1. In particular, the following $IC_{50}$ values (nM) were derived from inhibition of specific $GABA_B$ or $GABA_A$ binding to rat synaptic membranes for 3-(aminopropyl)methyl phosphinic acid (example 1) baclofen and 3-aminopropyl phosphinic acid.

Table I

| | $IC_{50}$ (nM) | |
|---|---|---|
| | $GABA_B$ | $GABA_A$ |
| Compound of Example 1 | 0.3 | 23,000 |
| 3-aminopropyl phosphinic acid | 1.0 | 420 |
| baclofen | 65 | - |

Potent selective $GABA_B$ agonists are expected to be of use in therapy in particular in the treatment of disorders of the central nervous system such as, multiple sclerosis, muscle relaxation in spinal spasticity, cerebral palsy, trigeminus neuralgia, pain and drug withdrawal symptoms, and other nervous disorders.

In addition, such compounds are expected to have utility in the treatment of cardiovascular disorders such as hypertension, gut motility disorders such as irritable bowel syndrome and as prokinetic agents.

When used in therapy, the compounds of the present invention are generally formulated into a pharmaceutical composition before administration. In a still further aspect there is therefore provided a pharmaceutical composition comprising a compound of structure (I) or a pharmaceutically acceptable salt thereof in association with a pharmaceutically acceptable carrier.

The pharmaceutical compositions of the present invention are prepared using standard pharmaceutical techniques and excipients for the preparation of suitable oral or parenteral compositions. For example, compositions for oral use can be in the form of liquid such as syrups, suspensions or emulsions, tablets, capsules and lozenges.

A liquid formulation will generally consist of a suspension or solution of the compound or pharmaceutically acceptable salt in a suitable liquid carrier(s) for example, ethanol, glycerine, non-aqueous solvent, for example polyethylene glycol, oils, or water with a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule; alternatively, a dispersion or suspension can be prepared using any suitable pharmaceutical carrier(s), for example aqueous gums, celluloses, silicates or oils and the dispersion or suspension then filled into a soft gelatin capsule.

Typical parenteral compositions consist of a solution or suspension of the compound or pharmaceutically acceptable salt in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

A typical suppository formulation comprises a compound of formula (I) or a pharmaceutically acceptable salt thereof which is active when administered in this way, with a binding and/or lubricating agent such as polymeric glycols, gelatins or cocoa butter or other low melting vegetable or synthetic waxes or fats.

Preferably the composition is in unit dose form such as a tablet or capsule.

Each dosage unit for oral administration contains preferably from 1 to 250 mg (and for parenteral administration contains preferably from 0.1 to 25 mg) of a compound of the formula (I) or a pharmaceutically acceptable salt thereof calculated as the free base.

The daily dosage regimen for an adult patient may be, for example, an oral dose of between 1 mg and 500 mg, preferably between 1 mg and 250 mg, or an intravenous, subcutaneous, or intramuscular dose of between 0.1 mg and 100 mg, preferably between 0.1 mg and 25 mg, of the compound of the formula (I) or a pharmaceutically acceptable salt thereof calculated as the free base, the compound being administered 1 to 4 times per day. Suitably the compounds will be administered for a period of continuous therapy for example for a week or

more.

Biological Data

Protocol for Binding Experiments

Preparation of Synaptic Membranes

Male rats were killed by decapitation. The brains were removed and homogenized immediately in 15 volumes ice-cold 0.32 M sucrose using a Potter-Elvehjem homogenizer filtered with a teflon pestle (radial clearance 0.25 mm). The homogenate was centrifuged at 1000 g to give a crude nuclear pellet ($P_1$) and a supernatant fraction enriched in myelin, synaptosomes and mitochrondria. The supernatant was collected and recentrifuged for 20 minutes at 20,000 g. The resulting pellet ($P_2$) was lysed by resuspension in distilled water and the mixture centrifuged for 20 minutes at 8,000 g. The supernatant was used to rinse off the upper layer of the pellet with the aid of a Pasteur pipette. The combined suspension was centrifuged for 20 minutes at 48,000 g. The final crude synaptic membranes were washed twice with distilled water and were either resuspended in buffer for further washing prior to use as fresh tissue or were stored frozen at -20°C.

Radioligand Binding Assay

All synaptic membranes were subjected to an extensive washing procedure to remove endogenous GABA and other possible inhibitory substances. Freshly prepared or frozen and thawed membranes were resuspended in the appropriate buffer (50 mM Tris-HCl pH 7.4 containing 2.5 mM $Ca^{++}$) and incubated under ambient conditions for 45 minutes before centrifugation at 7.500 g for 10 minutes. The tissue was washed a further 3 times by resuspension and recentrifugation with incubation periods of 15 minutes between spins. The final tissue was resuspended in fresh buffer for assay and transferred in 0.8 ml aliquots to microtubes (0.5 - 1.0 mg protein.tube).

[$^3$H]-GABA (10 nM final concentrations 65 Ci/mmol) was added to each tube with or without varying concentrations of unlabelled test compound (final volume 1.0 ml). All drugs were made up using either buffer solution or distilled water for subsequent dilution into incubation solution. Where additional compounds were required to be present throughout the incubation, these were added to the buffer solution containing the membranes, just prior to the addition of $^3$H-GABA. The final mixture was incubated under the required conditions (10 minutes at room temperature) and the assay was terminated by centrifugation at 7,500 g for 10 minutes. $GABA_B$ binding was determined by using $^3$H-GABA in the presence of isoguvacine (40 μM), a $GABA_A$ receptor ligand. $GABA_A$ binding was measured using $^3$H-GABA in the presence of baclofen (100 μM), a specific $GABA_B$ receptor ligand. Non-specific binding was obtained by incubation under $GABA_B$ conditions in the additional presence of 100 μM baclofen and under $GABA_A$ conditions in the additional presence of 100 μM isoguvacine. Following centrifugation the supernatant was aspirated off and the pellet rinsed twice superficially with ice- cold buffer solution.

The tissue was digested using 'Soluene-350' tissue solubilizer at 35°C and the test tube and its contents were transferred to a scintillation vial containing 400 μl of 0.2 M HCl to neutralize the 'Soluene'. The tritium content of each sample was estimated by liquid scintillation spectrometry.

Non-specific binding was subtracted from the total amount of radioactivity bound in the absence of any drug to yield the amount of ligand specifically bound. The amount of radioactivity specifically bound at a given concentration of non-radioactive drug was expressed as a percentage of the total ligand specifically bound.

The results obtained are presented in Table I referred to earlier.

Example 1

Preparation of 3-(aminopropyl)methylphosphinic acid

A. N-[3-(Ethoxymethylphosphinyl)] propyl phthalimide

Diethyl methlphosphonite prepared by the method of Hoffman, F.W. and Moore T.R. (JACS, 1985, 80, 1150-1154 (4.1 g, 0.03 mol), N-(3-bromopropyl)phthalimide (2.7 g, 0.01 mol) were placed in a 100 ml flask, covered with toluene (40 ml) and heated at reflux under $N_2$ for 17 hours. The mixture was cooled and the solvent removed in vacuo to give crude product. this was purified by flash chromatography on silica gel, eluting with mixtures of ethyl acetate/hexane followed by ethyl acetate/isopropanol to give product as a gum, yield, 1.1 g

(37%); 'HNMR ($CDCl_3$, 250 MHz), δ 1.31 (t, 3H, $CH_2\underline{CH_3}$), 1.46 (d, 3H, $CH_3P$), 1.80 (m, 2H, $CH_2$), 1.98 (m, 2H, $CH_2P$), 3.77 (t, 2H, $CH_2N$), 4,05 (q, 2H, $CH_2O$), 7.74, 7.86 (2 x q, 2 x 2H, ArH).

B. 3-(Aminopropyl)methylphosphinic acid

The above product (0.8 g, 0.003 mol) was placed in a 50 ml flask, covered with 6N hydrochloric acid (20 ml) and glacial acetic acid (2 ml) and heated at reflux for 3 hours. The mixture was cooled and the solvent removed in vacuo. The resulting residue was co-evaporated with water (3 times), ethanol (once) and toluene (once). The residue was taken up in dry methanol (5 ml), cooled to 0°C and treated with propylene oxide (1 ml) to give a white microcrystaline solid product yield, 0.16 g (40%), m.p. >300°C; 1HNMR ($D_2O$, 250 MHz), δ 1.24 (d, 3H, $\underline{CH_3}P$), 1.60 (m, 2H, $CH_2P$), 1.85 (m, 2H, $CH_2$), 3.05 (t, 2H, $CH_2N$),

| Anal: | $C_4H_{12}NO_2P$ | | |
|---|---|---|---|
| Found | : C, 34.92; | H, 8.71; | N, 9.94 |
| Calculated | : C, 35.04; | H, 8.82; | N, 10.22. |

## Claims

### Claims for the following Contracting States : AT, BE, CH, DE, FR, GB, IT, LI, LU, NL, SE

1. 3-(Aminopropyl)methyl phosphinic acid or a pharmaceutically acceptable salt thereof for use in therapy.

2. A pharmaceutical composition comprising 3-(aminopropyl)methyl phosphinic acid or a pharmaceutically acceptable salt thereof in association with a pharmaceutically acceptable carrier.

### Claims for the following Contracting States : GR, ES

1. A process for preparing a pharmaceutical composition which comprises bringing into association 3-(aminopropyl)methyl phosphinic acid or a pharmaceutically acceptable salt thereof, and a pharmaceutically acceptable carrier.

2. The use of 3-(aminopropyl)methyl phosphinic acid or a pharmaceutically acceptable salt thereof for the manufacture of a medicament for the treatment of disorders of the central nervous system.

## Patentansprüche

### Patentansprüche für folgende Vertragsstaaten: AT, BE, CH, DE, FR, GB, IT, LI, LU, NL, SE

1. 3-(Aminopropyl)methylphosphinsäure oder ein pharmazeutisch verträgliches Salz davon zu therapeutischer Verwendung.

2. Arzneimittel, umfassend 3-(Aminopropyl)methylphosphin- oder ein pharmazeutisch verträgliches Salz davon, zusammen mit einem pharmazeutisch verträglichen Träger.

### Patentansprüche für folgende Vertragsstaaten : GR, ES

1. Verfahren zur Herstellung eines Arzneimittels, das das Zusammenbringen von 3-(Aminopropyl)-methylphosphinsäure oder eines pharmazeutisch verträglichen Salzes davon mit einem pharmazeutisch verträglichen Träger umfaßt.

2. Verwendung von 3-(Aminopropyl)methylphosphinsäure oder einem pharmazeutisch verträglichen Salz davon zur Herstellung eines Arzneimittels zur Behandlung von Störungen des Zentralnervensystems.

## Revendications

### Revendications pour les Etats contractants suivants : AT, BE, CH, DE, FR, GB, IT, LI, LU, NL, SE

1. Acide 3-(aminopropyl)méthylphosphinique ou un de ses sels pharmaceutiquement acceptables pour l'emploi en thérapeutique.

2. Composition pharmaceutique comprenant de l'acide 3-(aminopropyl)méthylphosphinique ou un de ses sels pharmaceutiquement acceptables en association avec un véhicule pharmaceutiquement acceptable.

### Revendications pour les Etats contractants suivants : GR, ES

1. Procédé pour préparer une composition pharmaceutique qui comprend l'association de l'acide 3-(aminopropyl)méthylphosphinique ou d'un de ses sels pharmaceutiquement acceptables et d'un véhicule pharmaceutiquement acceptable.

2. Utilisation de l'acide 3-(aminopropyl)méthylphosphinique ou d'un de ses sels pharmaceutiquement acceptables pour la préparation d'un médicament pour le traitement de troubles du système nerveux central.